(19) Europäisches Patentamt
European Patent Office
Office européen des brevets

(11) Veröffentlichungsnummer: **0 473 980 A1**

(12) **EUROPÄISCHE PATENTANMELDUNG**

(21) Anmeldenummer: **91113561.4**

(22) Anmeldetag: **13.08.91**

(51) Int. Cl.[5]: **C07D 277/46**, C07D 285/08, //A01N43/78,A01N43/82

(30) Priorität: **25.08.90 DE 4026904**

(43) Veröffentlichungstag der Anmeldung: **11.03.92 Patentblatt 92/11**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Maurer, Fritz, Dr.**
**Roeberstrasse 8**
**W-5600 Wuppertal(DE)**
Erfinder: **Rohe, Lothar, Dr.**
**Damaschkeweg 75**
**W-5600 Wuppertal(DE)**
Erfinder: **Knops, Hans-Joachim, Dr.**
**Köpenickerstrasse 35**
**W-4019 Monheim(DE)**
Erfinder: **Kleefeld, Gerd, Dr.**
**Franziskusstrasse 27**
**W-4040 Neuss-Üdesheim(DE)**

(54) **Verfahren zur Herstellung von 3-Alkoxy-2-heteroazolylaminoacrylsäureestern.**

(57) Beschrieben wird ein neues Verfahren zur Herstellung von 3-Alkoxy-2-heteroazolylamino-acrylsäureestern der Formel (I)

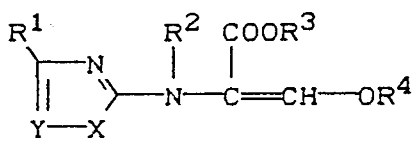

(I)

in welcher $R^1$, $R^2$, $R^3$, $R^4$, X und Y die in der Beschreibung angegebene Bedeutung haben, durch Umsetzung von Heteroazolylaminoessigsäuren der Formel (II)

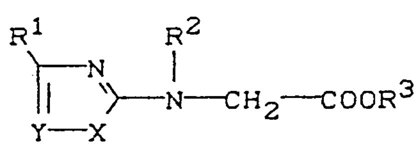

(II)

mit Ameisensäureestern und anschließender Alkylierung.

Ferner werden neue Zwischenprodukte bzw. Ausgangsprodukte sowie neue Verfahren zur Herstellung neuer und bekannter Zwischenprodukte beschrieben.

EP 0 473 980 A1

Die Erfindung betrifft ein neues Verfahren zur Herstellung von bekannten fungizid wirksamen 3-Alkoxy-2-heteroazolylamino-acrylsäureestern sowie ein neues Verfahren zur Herstellung der als Zwischenprodukte benötigten Heteroazolylaminoessigsäureester.

Es ist bekannt, daß man 3-Alkoxy-2-heteroazolylamino-acrylsäureester ausgehend von entsprechenden Heteroazolylaminoessigsäureestern erhält, wenn man zunächst durch Umsetzung mit Natriumhydrid und Ameisensäuremethylester in Dimethylformamid entsprechende 3-Hydroxy-2-heteroazolylamino-acrylsäureester herstellt und diese mit Alkylierungsmitteln in Gegenwart von Säureakzeptoren umsetzt (vergleiche DE-OS 38 07 232 / EP-A 331 966 / LeA 25 908; DE-OS 39 05 119 / LeA 26 672; DE-OS 39 10 358 / LeA 26 757).

Das bekannte Verfahren ist für industrielle Belange nicht geeignet; insbesondere stellt die Verwendung von Natriumhydrid - aus Kostengründen und unter sicherheitstechnischen Aspekten - ein schwerwiegendes Hindernis für die Übertragung in einen größeren Maßstab dar. Zudem sind die Ausbeuten - über beide Reaktionsstufen - bei der bekannten Verfahrensweise nicht zufriedenstellend.

Es wurde nun gefunden, daß man 3-Alkoxy-2-heteroazolylamino-acrylsäureesterder allgemeinen Formel (I)

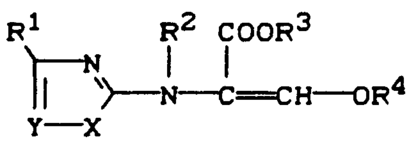

(I)

in welcher

$R^1$ für jeweils gegebenenfalls substituiertes Aryl oder Heteroaryl steht,

$R^2$ für Wasserstoff oder Alkyl steht,

$R^3$ für Alkyl steht,

$R^4$ für Alkyl steht,

X für Sauerstoff oder Schwefel steht und

Y für Stickstoff oder die Gruppierung $C-R^5$ steht, wobei

$R^5$ für Wasserstoff, Halogen, Cyano, Formyl, Carboxy, Nitro oder für jeweils gegebenenfalls substituiertes Alkyl, Alkoxy oder Alkylthio oder für Alkoxycarbonyl steht,

in guten Ausbeuten und in hoher Reinheit erhält, wenn man Heteroazolylaminoessigsäureester der allgemeinen Formel (II)

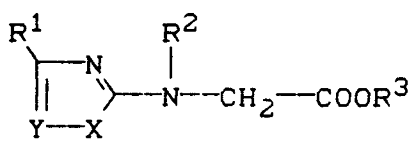

(II)

in welcher

$R^1$, $R^2$, $R^3$, X und Y die oben angegebene Bedeutung haben,

mit Ameisensäureestern der allgemeinen Formel (III)

$HCOOR^6$ (III)

in welcher

$R^6$ für Alkyl steht,

in Gegenwart eines Alkalimetallalkanolats und gegebenenfalls in Gegenwart eines Verdünnungsmittels bei Temperaturen von 0 °C bis 100 °C umsetzt und das hierbei erhältliche Produkt der Formel (IV)

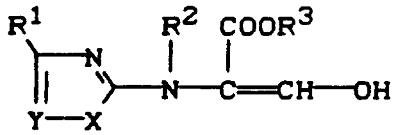

(IV)

in situ (ohne Zwischenisolierung) mit einem Alkylierungsmittel der allgemeinen Formel (V)

$X^1—R^4$ (V)

in welcher

$R^4$ die oben angegebene Bedeutung hat und

$X^1$ für Halogen oder die Gruppierung $-O-SO_2-O-R^4$ steht,

bei Temperaturen von 0 °C bis 100 °C umsetzt.

Überraschenderweise können nach dem erfindungsgemäßen Verfahren die 3-Alkoxy-2-heteroazolylamino-acrylsäureester der Formel (I) in höheren Ausbeuten und in besserer Qualität als nach der bekannten Verfahrensweise erhalten werden.

Im Gegensatz zum bekannten Verfahren ist das erfindungsgemäße Verfahren problemlos in einen größeren Maßstab übertragbar; auf die sonst erforderliche chromatographische Reinigung des Produktes kann beim erfindungsgemäßen Verfahren verzichtet werden.

Nach dem erfindungsgemäßen Verfahren erhält man die Verbindungen der Formel (I) als Gemische der jeweils möglichen E- und Z-Isomeren in wechselnder Zusammensetzung:

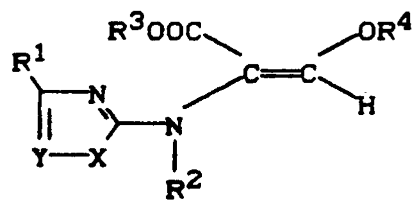

(I)/E

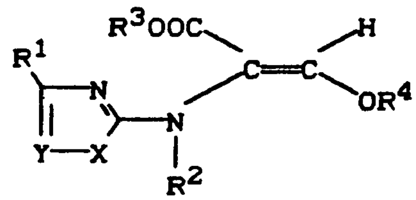

(I)/Z

Verwendet man beispielsweise N-Methyl-N-(3-phenyl-1,2,4-thiadiazol-5-yl)-aminoessigsäure-methylester, Ameisensäuremethylester und Natrium-methanolat sowie in der Folgestufe Methylbromid als Ausgangsstoffe, so kann der Reaktionsablauf beim erfindungsgemäßen Verfahren durch das folgende Formelschema wiedergegeben werden:

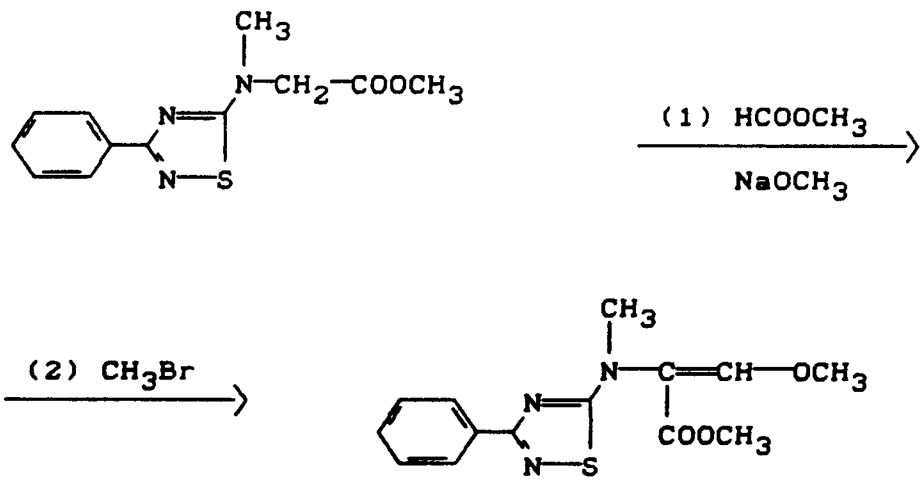


Nach dem erfindungsgemäßen Verfahren werden vorzugsweise Verbindungen der Formel (I) hergestellt, in welcher

$R^1$ für jeweils gegebenenfalls substituiertes Aryl oder Heteroaryl mit jeweils bis zu 10 Kohlenstoffatomen und gegebenenfalls 1 bis 4 Stickstoffatomen und/oder einem Sauerstoff- oder Schwefelatom als Heteroatom(en) steht, wobei als bevorzugte Substituenten ausgewählt sind:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Aryloxyalkyl oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 4 Kohlenstoffatomen im geradkettigen oder verzweigten Alkylteil;

$R^2$ für Wasserstoff oder für geradkettiges oder verzweigtes Alkyl mit 1 bis 6 Kohlenstoffatomen steht,

$R^3$ für geradkettiges oder verzweigtes Alkyl mit 1 bis 6 Kohlenstoffatomen steht,

$R^4$ für geradkettiges oder verzweigtes Alkyl mit 1 bis 6 Kohlenstoffatomen steht,

X für Sauerstoff oder Schwefel steht und

Y für Stickstoff oder die Gruppierung C-$R^5$ steht, wobei

$R^5$ für Wasserstoff, Halogen, Cyano, Formyl, Carboxy, Nitro oder für jeweils geradkettiges oder verzweigtes Alkyl, Halogenalkyl, Alkoxy, Halogenalkoxy, Alkylthio oder Halogenalkylthio mit jeweils 1 bis 4 Kohlenstoffatomen und gegebenenfalls 1 bis 9 Halogenatomen oder für geradkettiges oder verzweigtes Alkoxycarbonyl mit bis zu 4 Kohlenstoffatomen steht.

Insbesondere werden nach dem erfindungsgemäßen Verfahren Verbindungen der Formel (I) hergestellt, in welcher

$R^1$ für jeweils gegebenenfalls einfach bis dreifach substituiertes Phenyl, Naphthyl, Pyridyl, Furyl oder Thienyl steht, wobei als besonders bevorzugte Substituenten ausgewählt sind: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl,

Methoxy, Ethoxy, Methylthio, Trifluormethyl, Difluormethoxy, Trifluormethoxy und/oder Trifluormethylthio substituiertes Phenyl, Benzyl, Phenoxy, Phenoxymethyl oder Benzyloxy,

$R^2$ für Wasserstoff, Methyl, Ethyl, Propyl, Isopropyl, Butyl oder Isobutyl (insbesondere für Methyl) steht,

$R^3$ für Methyl, Ethyl, Propyl, Isopropyl, Butyl oder Isobutyl (insbesondere für Methyl) steht,

$R^4$ für Methyl, Ethyl, Propyl, Isopropyl, Butyl oder Isobutyl (insbesondere für Methyl) steht,

X für Schwefel steht und

Y für Stickstoff oder die Gruppierung C-$R^5$ steht, wobei

$R^5$ für Wasserstoff, Fluor, Chlor, Brom, Iod, Cyano, Formyl, Methyl, Difluormethyl, Dichlormethyl, Trifluormethyl, Chlordifluormethyl, Methoxy oder Methoxycarbonyl (insbesondere für Chlor oder Brom) steht.

Die beim erfindungsgemäßen Verfahren zur Herstellung von Verbindungen der Formel (I) als Ausgangsstoffe zu verwendenden Heteroazolylaminoessigsäureester sind durch die Formel (II) allgemein definiert.

In Formel (II) haben $R^1$, $R^2$, $R^3$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, X und Y angegeben wurden.

Die Heteroazolylaminoessigsäureester der Formel (II) sind bekannt und/oder können nach an sich bekannten Verfahren hergestellt werden (vergleiche DE-OS 38 07 232; EP-A 331 966; DE-OS 39 05 119; DE-OS 39 10 358; Herstellungsbeispiele). Ein erfinderisches neues Verfahren zu ihrer Herstellung ist Gegenstand dieser Patentanmeldung (siehe unten).

Die beim erfindungsgemäßen Verfahren weiter als Ausgangsstoffe zu verwendenden Ameisensäureester sind durch die Formel (III) allgemein definiert. In Formel (III) steht $R^6$ vorzugsweise für geradkettiges oder verzweigtes Alkyl mit 1 bis 6 Kohlenstoffatomen, insbesondere für Methyl oder Ethyl.

Die Ameisensäureester der Formel (III) sind bekannte organische Synthesechemikalien.

Das erfindungsgemäße Verfahren wird unter Verwendung von Alkalimetallalkanolaten durchgeführt. Vorzugsweise werden Natrium- oder Kaliumalkanolate mit 1 bis 6 Kohlenstoffatomen im geradkettigen oder verzweigten Alkanolatrest eingesetzt. Besonders bevorzugt werden Natriummethanolat oder -ethanolat (insbesondere Natriummethanolat) sowie Kalium-methanolat oder -ethanolat.

Das erfindungsgemäße Verfahren wird vorzugsweise unter Verwendung von Verdünnungsmitteln durchgeführt. Als Verdünnungsmittel kommen dabei praktisch alle inerten organischen Lösungsmittel infrage. Hierzu gehören beispielsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Verdünnungsmittel bevorzugt werden aprotisch polare Solventien aus der Reihe der Carbonsäureamide und Harnstoffe, wie z. B. Dimethylformamid, Dimethylacetamid, Diethylformamid, Dipropylformamid, Dibutylformamid, N-Methylpyrrolidon und Tetramethylharnstoff.

Die Reaktionstemperaturen können bei dem erfindungsgemäßen Verfahren in einem größeren Bereich variiert werden. Im allgemeinen arbeitet man bei Temperaturen von 0°C bis 100°C, vorzugsweise bei Temperaturen von 0°C bis 60°C.

Das erfindungsgemäße Verfahren wird im allgemeinen unter Normaldruck durchgeführt. Es ist jedoch auch möglich, unter erhöhtem oder vermindertem Druck - im allgemeinen von 10 hPa bis 10 000 hPa - zu arbeiten.

Zur Durchführung des erfindungsgemäßen Verfahrens setzt man auf 1 Mol Heteroazolylaminoessigsäureester der Formel (II) im allgemeinen von 5 bis 50 Mol, vorzugsweise von 10 bis 30 Mol, Ameisensäureester der Formel (III), von 1 bis 4 Mol, vorzugsweise von 1,5 bis 3 Mol, Alkalimetallalkanolat, und in der Folgestufe von 1 bis 4 Mol, vorzugsweise von 1,5 bis 3 Mol, Alkylierungsmittel der Formel (V) ein.

In einer bevorzugten Ausführungsform des erfindungsgemäßen Verfahrens wird eine Mischung aus einem Heteroazolylaminoessigsäureester der Formel (II), einem Ameisensäureester der Formel (III) und einem Verdünnungsmittel vorgelegt, ein Alkalimetallalkanolat wird langsam dazu gegeben und das Gemisch bis zum Ablauf der ersten Reaktionsstufe gerührt. Wenn das Produkt der Formel (IV) nahezu quantitativ entstanden ist, wird ein Alkylierungsmittel der Formel (V) langsam eindosiert und das Reaktionsgemisch bis zum Ende der Umsetzung gerührt.

Die Aufarbeitung kann nach üblichen Methoden durchgeführt werden. Beispielsweise wird, gegebenenfalls nach Einengen, mit Wasser und einem mit Wasser praktisch nicht mischbaren organischen Lösungsmittel, wie z. B. Petrolether oder Essigsäureethylester, gegebenenfalls in Gegenwart eines Puffermittels, wie z. B. Natriumhydrogencarbonat, verrührt oder geschüttelt. Soweit das Produkt der Formel (I) hierbei kristallin anfällt, kann es durch Absaugen isoliert werden. Andernfalls wird die organische Phase, gegebenenfalls nach Vereinigen mit Nachextrakten, mit Wasser gewaschen, getrocknet und filtriert. Das nach Abdestillieren des Lösungsmittels verbleibende Rohprodukt kann auf übliche Weise, z. B. durch Umkristallisation gereinigt werden.

Gegenstand der vorliegenden Patentanmeldung ist auch ein neues Verfahren zur Herstellung von Heteroazolylaminoessigsäureestern der Formel (II), welche als Zwischenprodukte zur Herstellung der Verbindungen der Formel (I) benötigt werden.

Es ist bekannt, daß man Heteroazolylaminoessigsäureester erhält, wenn man Halogenheteroazole mit Aminoessigsäureestern oder Aminoheteroazole mit Halogenessigsäureestern umsetzt (vergleiche DE-OS 38 07 232 / EP-A 331 966 / LeA 25 908; DE-OS 39 10 358 / LeA 26 757). Nach beiden Verfahren werden die Heteroazolylaminoessigsäureester in unbefriedigenden Ausbeuten und in ungenügender Qualität erhalten.

Es wurde nun gefunden, daß man Heteroazolylaminoessigsäureester der allgemeinen Formel (II)

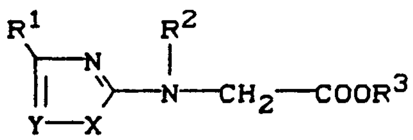

(II)

in welcher

$R^1$ für jeweils gegebenenfalls substituiertes Aryl oder Heteroaryl steht,

$R^2$ für Wasserstoff oder Alkyl steht,

$R^3$ für Alkyl steht,

X für Sauerstoff oder Schwefel steht und

Y für Stickstoff oder die Gruppierung C-$R^5$ steht, wobei

$R^5$ für Wasserstoff, Halogen, Cyano, Formyl, Carboxy, Nitro oder für jeweils gegebenenfalls substituiertes Alkyl, Alkoxy oder Alkylthio oder für Alkoxycarbonyl steht,

in guten Ausbeuten und in hoher Reinheit erhält, wenn man Heteroazolylaminoessigsäuren der allgemeinen Formel (VI)

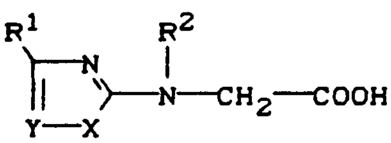

(VI)

in welcher

$R^1$, $R^2$, X und Y die oben angegebene Bedeutung haben,

mit Alkoholen der allgemeinen Formel (VII)

$HOR^3$ (VII)

in welcher

$R^3$ die oben angegebene Bedeutung hat,

in Gegenwart saurer Veresterungskatalysatoren bei Temperaturen von 0 °C bis 120 °C umsetzt.

Überraschenderweise können nach diesem Verfahren die Heteroazolylaminoessigsäureester der Formel (II) in nahezu quantitativer Ausbeute und in sehr guter Qualität erhalten werden.

Verwendet man beispielsweise N-Methyl-N-(3-phenyl-1,2,4-thiadiazol-5-yl)-aminoessigsäure und Methanol als Ausgangsstoffe, so kann der Reaktionsablauf durch das folgende Formelschema wiedergegeben werden:

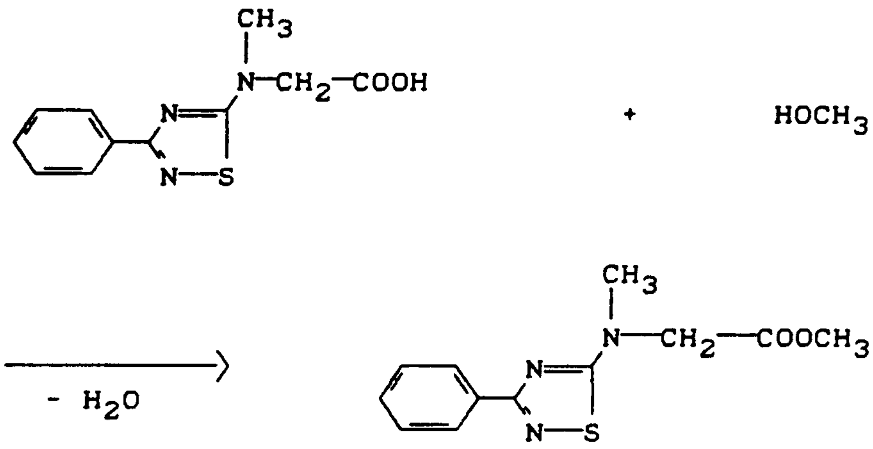


Die beim erfindungsgemäßen Verfahren zur Herstellung von Verbindungen der Formel (II) als Ausgangsstoffe zu verwendenden Heteroazolylaminoessigsäuren sind durch die Formel (VI) allgemein definiert.

In Formel (VI) haben $R^1$, $R^2$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, X und Y angegeben wurden.

Die Heteroazolylaminoessigsäuren der Formel (VI) sind noch nicht aus der Literatur bekannt und sind Gegenstand der vorliegenden Patentanmeldung.

Man erhält die neuen Heteroazolylaminoessigsäuren der Formel (VI)

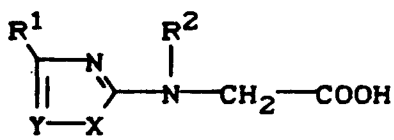

(VI)

in welcher
- $R^1$ für jeweils gegebenenfalls substituiertes Aryl oder Heteroaryl steht,
- $R^2$ für Wasserstoff oder Alkyl steht,
- X für Sauerstoff oder Schwefel steht und
- Y für Stickstoff oder die Gruppierung C-$R^5$ steht, wobei
- $R^5$ für Wasserstoff, Halogen, Cyano, Formyl, Carboxy, Nitro oder für jeweils gegebenenfalls substituiertes Alkyl, Alkoxy oder Alkylthio oder für Alkoxycarbonyl steht,

wenn man Aminoheteroazole der allgemeinen Formel (VIII)

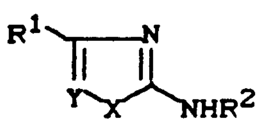

(VIII)

in welcher
$R^1$, $R^2$, X und Y die oben angegebene Bedeutung haben,
mit Halogenessigsäure-Salzen der allgemeinen Formel (IX)

$X^2-CH_2-COOM$ (IX)

in welcher
- M für ein Alkalimetall steht und
- $X^2$ für Halogen steht,

in Gegenwart eines Säureakzeptors und in Gegenwart eines Verdünnungsmittels bei Temperaturen von 0 ° C bis 100 ° C umsetzt (vergleiche die Herstellungsbeispiele).

Als Säureakzeptoren werden hierbei vorzugsweise Alkalimetallhydroxide, wie z. B. Natrium- oder Kaliumhydroxid, oder Alkalimetallalkoholate, wie z. B. Natrium- oder Kalium-tert-butylat oder -tert-amylat, verwendet.

Als Verdünnungsmittel werden vorzugsweise inerte organische Solventien, wie z. B. Hexan, Cyclohexan, Methylcyclohexan, Toluol, Xylol, Tetrahydrofuran, Dioxan, Methyl-tert-butylether, Methyl-tert-amylether, Dimethylsulfoxid, Dimethylformamid, Dimethylacetamid, N-Methylpyrrolidon, Dibutylformamid und Tetramethylharnstoff, verwendet.

Die als Ausgangsstoffe zu verwendenden Aminoheteroazole sind durch die Formel (VIII) allgemein definiert. In Formel (VIII) haben $R^1$, $R^2$, X und Y vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^1$, $R^2$, X und Y angegeben wurde.

Die Aminoheteroazole der Formel (VIII) sind bekannt und/oder können nach an sich bekannten Verfahren hergestellt werden (vergleiche Org. Reactions, Vol. 6, 367-409; Heterocycles 23 (1985), 2645-2649; J. Heterocycl. Chem. 22 (1985), 1621-1630; vergleiche auch oben angegebenen Stand der Technik und die Herstellungsbeispiele).

Die weiter als Ausgangsstoffe zu verwendenden Halogenessigsäure-Salze sind durch die Formel (IX) allgemein definiert. In Formel (IX) steht M vorzugsweise für Lithium, Natrium oder Kalium, insbesondere für Natrium, und $X^2$ steht vorzugsweise für Chlor, Brom oder Iod, insbesondere für Chlor.

Die Halogenessigsäure-Salze der Formel (IX) sind bekannte Synthesechemikalien.

Die beim erfindungsgemäßen Verfahren zur Herstellung der Heteroazolylaminoessigsäureester der Formel (II) weiter als Ausgangsstoffe zu verwendenden Alkohole sind durch die Formel (VII) allgemein definiert. In Formel (VII) hat $R^3$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^3$ angegeben wurde.

Die Alkohole der Formel (VII) sind bekannte Synthesechemikalien.

Das erfindungsgemäße Verfahren zur Herstellung der Heteroazolylaminoessigsäureester der Formel (II) wird unter Verwendung von sauren Veresterungskatalysatoren durchgeführt. Als solche sind vor allem anorganische Mineralsäuren, wie z. B. Salzsäure, Schwefelsäure oder Phosphorsäure, oder auch organische Sulfonsäuren, wie z. B. Methansulfonsäure, Benzolsulfonsäure oder p-Toluolsulfonsäure, geeignet.

Die Reaktionstemperaturen können beim erfindungsgemäßen Verfahren zur Herstellung der Heteroazolylaminoessigsäureester der Formel (II) in einem größeren Bereich variiert werden. Im allgemeinen arbeitet man bei Temperaturen von 0°C bis 120°C, vorzugsweise bei Temperaturen von 20°C bis 90°C.

Das erfindungsgemäße Verfahren zur Herstellung der Heteroazolylaminoessigsäureester der Formel (II) wird im allgemeinen bei atmosphärischem Druck durchgeführt. Es ist jedoch auch möglich, unter erhöhtem oder vermindertem Druck - im allgemeinen von 50 hPa bis 5000 hPa - zu arbeiten.

Zur Durchführung des erfindungsgemäßen Verfahrens zur Herstellung der Heteroazolylaminoessigsäureester der Formel (II) setzt man auf 1 Mol Heteroazolylaminoessigsäure der Formel (VI) im allgemeinen 20 bis 200 Mol, vorzugsweise 50 bis 100 Mol, Alkohol der Formel (VII) und 1 g bis 50 g, vorzugsweise 5 g bis 15 g, eines sauren Veresterungskatalysators ein.

Im allgemeinen werden die Ausgangsstoffe der Formeln (VI) und (VII) mit dem Veresterungskatalysator bei Raumtemperatur vermischt und dann bei erhöhter Temperatur bis zum Ende der Umsetzung gerührt.

Die Aufarbeitung kann auf übliche Weise durchgeführt werden. Beispielsweise wird eingeengt, der Rückstand mit Wasser, welches gegebenenfalls ein Puffermittel, wie z. B. Natriumhydrogencarbonat enthält, und einem mit Wasser praktisch nicht mischbarem organischem Lösungsmittel, wie z. B. Methylenchlorid, geschüttelt, die organische Phase dann gegebenenfalls mit Wasser gewaschen, getrocknet und filtriert. Vom Filtrat wird das Lösungsmittel unter vermindertem Druck sorgfältig abdestilliert. Das als Rückstand erhaltene Rohprodukt kann auf übliche Weise gereinigt werden (vergleiche die Herstellungsbeispiele).

Gegenstand der vorliegenden Patentanmeldung ist neben den oben beschriebenen Verfahren zur Herstellung der 3-Alkoxy-2-heteroazolylamino-acrylsäureester der Formel (I) und der Heteroazolylaminoessigsäureester der Formel (II) die Kombination der oben im einzelnen beschriebenen Verfahrensschritte ausgehend von den Aminoheteroazolen der Formel (VIII) und den Halogenessigsäure-Salzen der Formel (IX) gemäß dem folgenden Reaktionsschema; nach diesem Verfahrensweg können die Verbindungen der Formel (I) wesentlich einfacher und in besseren Ausbeuten sowie höherer Qualität als nach den oben angegebenen bekannten Verfahren hergestellt werden.

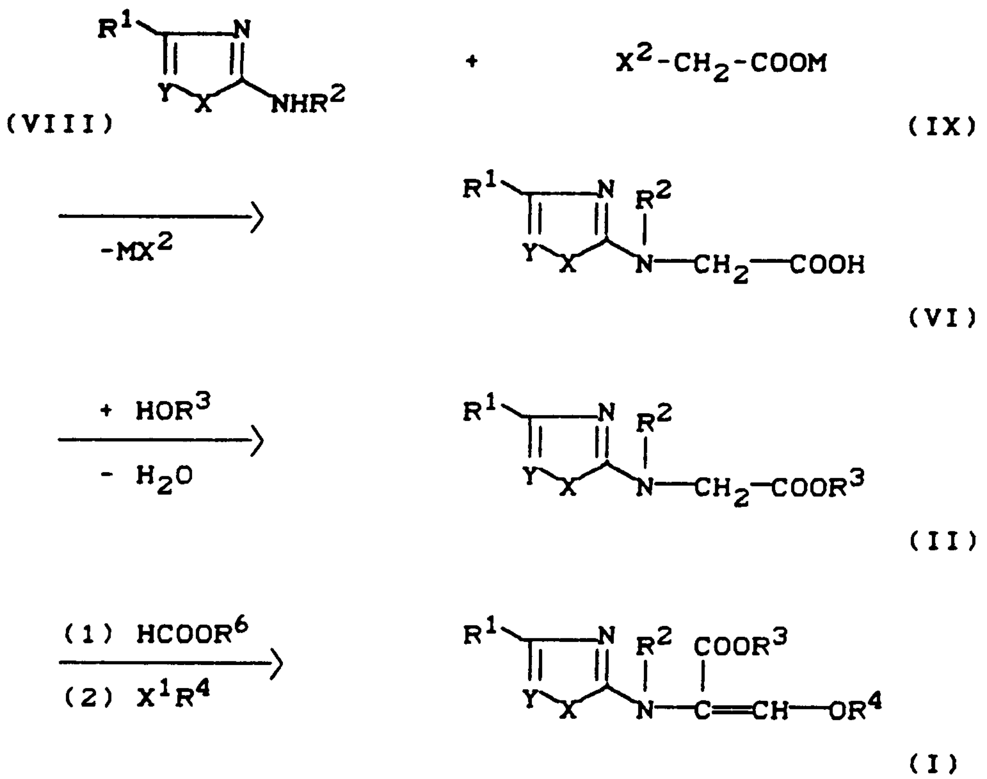


(VIII) (IX) (VI) (II) (I)

Die nach den erfindungsgemäßen Verfahren herzustellenden Verbindungen der Formel (I) sind, wie bereits oben erwähnt, fungizid wirksam; sie können beispielsweise im Pflanzenschutz zur Bekämpfung von Pilzkrankheiten verwendet werden (vergleiche DE-OS 38 07 232; DE-OS 39 05 119; DE-OS 39 10 358).

Herstellungsbeispiele:

Verbindungen der Formel (I):

Beispiel I-1

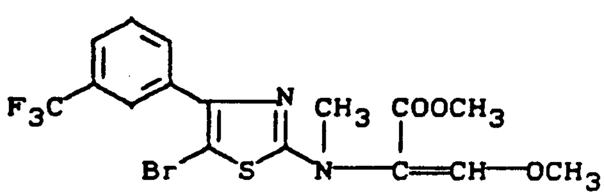

Eine Mischung aus 61,4 g (0,15 Mol) N-Methyl-N-(5-brom-4-(3-trifluormethyl-phenyl)-thiazol-2-yl)-aminoessigsäure-methylester, 180 ml (2,9 Mol) Ameisensäuremethylester und 150 ml Dimethylformamid wird auf 2°C bis 5°C abgekühlt und unter Rühren mit insgesamt 17,9 g (0,33 Mol) Natrium-methanolat portionsweise versetzt. Die Mischung wird 18 Stunden bei 2°C bis 5°C gerührt. Dann werden bei der gleichen Temperatur 42,2 g (0,335 Mol) Dimethylsulfat innerhalb von 30 Minuten eindosiert; das Gemisch wird 5 Stunden bei 2°C bis 5°C und weitere 12 Stunden bei 20°C gerührt. Anschließend wird unter vermindertem Druck eingeengt und der Rückstand mit 1 Liter 5%iger Natriumhydrogencarbonat-Lösung verrührt. Nach Zugabe von 300 ml Petrolether wird das kristallin angefallene Produkt durch Absaugen isoliert.

Man erhält 51 g (75 % der Theorie) 3-Methoxy-2-(N-methyl-N-(5-brom-4-(3-trifluormethylphenyl)-thiazol-2-yl)-amino)-acrylsäure-methylester vom Schmelzpunkt 87°C.

Beispiel I-2

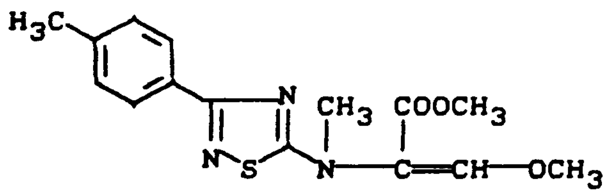

Eine Mischung aus 115 g (0,415 Mol) N-Methyl-N-(3-(4-methyl-phenyl)-1,2,4-thiadiazol-5-yl)-aminoessigsäuremethylester, 252 g (4,2 Mol) Ameisensäuremethylester und 300 ml Dimethylformamid wird unter Rühren innerhalb von 20 Minuten mit 44,8 g (0,83 Mol) Natriummethylat protionsweise versetzt und die Mischung wird 4 Stunden bei 20 ° C gerührt. Dann werden 105 g (0,83 Mol) Dimethylsulfat zugetropft und das Reaktionsgemisch wird 15 Stunden bei 20 ° C gerührt. Anschließend wird mit Wasser verdünnt, die organische Phase abgetrennt, die wässrige Phase dreimal mit je 150 ml Essigsäureethylester extrahiert, die vereinigten organischen Phasen mit Wasser gewaschen, mit Natriumsulfat getrocknet und filtriert. Vom Filtrat wird das Lösungsmittel unter vermindertem Druck sorgfältig abdestilliert und das zurückbleibende Rohprodukt (132 g) durch Kristallisation aus Cyclohexan / Essigsäureethylester (Vol. 3:1) gereinigt.
Man erhält 88 g (67 % der Theorie) 3-Methoxy-2-(N-methyl-N-(3-(4-methyl-phenyl)-1,2,4-thiadiazol-5-yl)-amino)-acrylsäure-methylester vom Schmelzpunkt 102 ° C.

Analog zu den Beispielen I-1 und I-2 sowie nach der allgemeinen Beschreibung des erfindungsgemäßen Verfahrens können beispielsweise auch die in der nachstehenden Tabelle 1 aufgeführten Verbindungen der Formel (1) hergestellt werden.

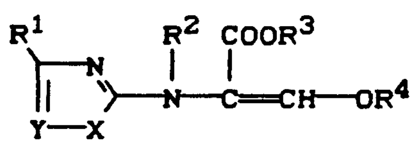

(I)

**Tabelle 1:** Beispiele für die erfindungsgemäß herzustellenden Verbindungen der Formel (I)

| Beisp.-Nr. | $R^1$ | $R^2$ | $R^3$ | $R^4$ | X | Y | Ausbeute (% d.Th.) | Schmelzpunkt (°C) |
|---|---|---|---|---|---|---|---|---|
| I-3 | 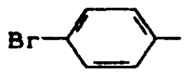 | $CH_3$ | $CH_3$ | $CH_3$ | S | C-Br | 60 | amorph |
| I-4 | 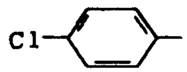 | $CH_3$ | $CH_3$ | $CH_3$ | S | C-Br | 76 | 97 |
| I-5 | 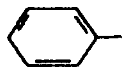 | $CH_3$ | $CH_3$ | $CH_3$ | S | N | | amorph |
| I-6 | 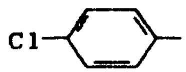 | $CH_3$ | $CH_3$ | $CH_3$ | S | N | | 106 |
| I-7 | 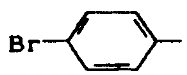 | $CH_3$ | $CH_3$ | $CH_3$ | S | N | | 91 |
| I-8 | 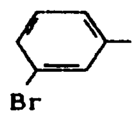 | $CH_3$ | $CH_3$ | $CH_3$ | S | C-Cl | 51 | 95 |
| I-9 | 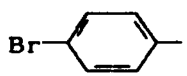 | $CH_3$ | $CH_3$ | $CH_3$ | S | CH | | |
| I-10 | 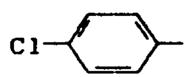 | $CH_3$ | $CH_3$ | $CH_3$ | S | C-Cl | | |

**Tabelle 1:** Beispiele für die erfindungsgemäß herzustellenden Verbindungen der Formel (I) (Fortsetzung)

| Beisp.-Nr. | $R^1$ | $R^2$ | $R^3$ | $R^4$ | X | Y | Ausbeute (% d.Th.) | Schmelzpunkt (°C) |
|---|---|---|---|---|---|---|---|---|
| I-11 | 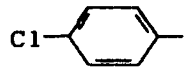 | $CH_3$ | $CH_3$ | $CH_3$ | S | CH | | |
| I-12 | 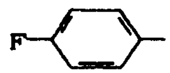 | $CH_3$ | $CH_3$ | $CH_3$ | S | C-Br | | |
| I-13 | 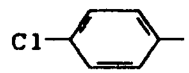 | $CH_3$ | $CH_3$ | $CH_3$ | S | $C-CH_3$ | | |
| I-14 | 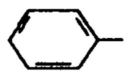 | $CH_3$ | $CH_3$ | $CH_3$ | S | C-Br | | |
| I-15 | 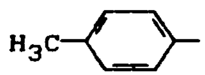 | $CH_3$ | $CH_3$ | $CH_3$ | S | C-H | 82 | 93 |
| I-16 | 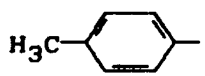 | $CH_3$ | $CH_3$ | $CH_3$ | S | C-Br | | |
| I-17 | 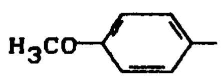 | $CH_3$ | $CH_3$ | $CH_3$ | S | CH | 83 | 120 |
| I-18 | 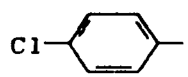 | $C_2H_5$ | $CH_3$ | $CH_3$ | S | C-Cl | | |

**Tabelle 1:** **Beispiele für die erfindungsgemäß herzustellenden Verbindungen der Formel (I) (Fortsetzung)**

| Beisp.-Nr. | $R^1$ | $R^2$ | $R^3$ | $R^4$ | X | Y | Ausbeute (% d.Th.) | Schmelzpunkt (°C) |
|---|---|---|---|---|---|---|---|---|
| I-19 | 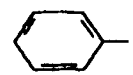 | $CH_3$ | $C_2H_5$ | $C_2H_5$ | S | C-Cl | | |
| I-20 | 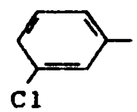 | $CH_3$ | $CH_3$ | $CH_3$ | S | C-Cl | | |
| I-21 | 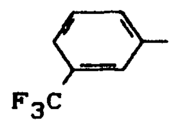 | $CH_3$ | $CH_3$ | $CH_3$ | S | C-Cl | | |
| I-22 | 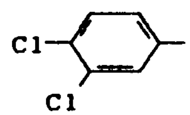 | $CH_3$ | $CH_3$ | $CH_3$ | S | CH | | |
| I-23 | 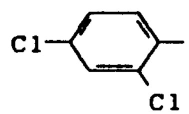 | $CH_3$ | $CH_3$ | $CH_3$ | S | CH | | |
| I-24 | 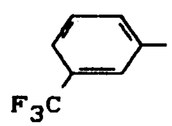 | $CH_3$ | $CH_3$ | $CH_3$ | S | N | | |
| I-25 | 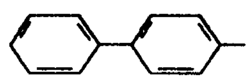 | $CH_3$ | $CH_3$ | $CH_3$ | S | N | | |

Verbindungen der Formel (II):

Beispiel II-1

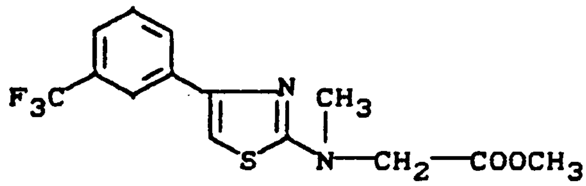

Eine Mischung aus 79,1 g (0,25 Mol) N-Methyl-N-(4-(3-trifluormethyl-phenyl)-thiazol-2-yl)-aminoessigsäure, 600 ml Methanol und 1,5 ml konzentrierte Schwefelsäure wird 30 Stunden unter Rückfluß zum Sieden erhitzt. Dann wird das überschüssige Methanol im Wasserstrahlvakuum abdestilliert und der Rückstand in 300 ml Methylenchlorid aufgenommen; diese Lösung wird dann mit 300 ml gesättigter Natriumhydrogencarbonat-Lösung und dann mit Wasser gewaschen, mit Natriumsulfat getrocknet und filtriert. Das Filtrat wird im Wasserstrahlvakuum eingeengt, der Rückstand mit Wasser verrührt und das kristallin angefallene Produkt durch Absaugen isoliert.

Man erhält 79,9 g (97 % der Theorie) N-Methyl-N-(4-(3-trifluormethyl-phenyl)-thiazol-2-yl)-aminoessigsäuremethylester vom Schmelzpunkt 115 °C.

Analog Beispiel II-1 können beispielsweise auch die in der nachstehenden Tabelle 2 aufgeführten Verbindungen der Formel (II) hergestellt werden.

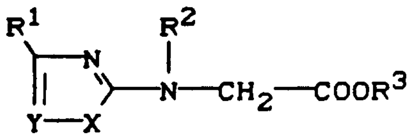

(II)

<u>Tabelle 2:</u> Beispiele für die erfindungsgemäß herzustellenden Verbindungen der Formel (II)

| Beisp.-Nr. | $R^1$ | $R^2$ | $R^3$ | X | Y | Ausbeute (% d.Th.) | Schmelzpunkt (°C) |
|---|---|---|---|---|---|---|---|
| II-2 | 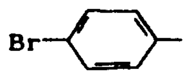 | $CH_3$ | $CH_3$ | S | CH | 97 | 100 |
| II-3 | 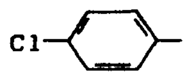 | $CH_3$ | $CH_3$ | S | CH | 96 | 88 |
| II-4 | 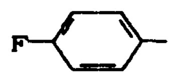 | $CH_3$ | $CH_3$ | S | CH | | |
| II-5 | 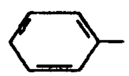 | $CH_3$ | $CH_3$ | S | CH | 98 | $n_D^{24}$ : 1,6088 |
| II-6 | 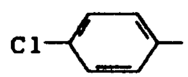 | $C_2H_5$ | $CH_3$ | S | CH | | |
| II-7 | 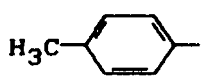 | $CH_3$ | $CH_3$ | S | CH | 96 | 84 |
| II-8 | 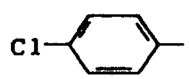 | $CH_3$ | $C_2H_5$ | S | CH | | |
| II-9 | 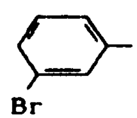 | $CH_3$ | $CH_3$ | S | CH | 88 | 91 |

**Tabelle 2:** Beispiele für die erfindungsgemäß herzustellenden Verbindungen der Formel (II) (Fortsetzung)

| Beisp.-Nr. | $R^1$ | $R^2$ | $R^3$ | X | Y | Ausbeute (% d.Th.) | Schmelzpunkt (°C) |
|---|---|---|---|---|---|---|---|
| II-10 | 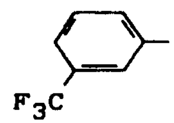 | $CH_3$ | $CH_3$ | S | CH | | |
| II-11 | 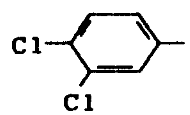 | $CH_3$ | $CH_3$ | S | CH | | |
| II-12 | 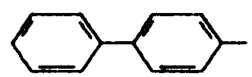 | $CH_3$ | $CH_3$ | S | CH | | |
| II-13 | 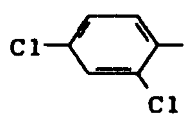 | $CH_3$ | $CH_3$ | S | CH | | |
| II-14 | 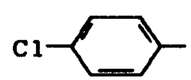 | $CH_3$ | $CH_3$ | S | C-Cl | | |
| II-15 | 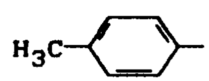 | $CH_3$ | $CH_3$ | S | C-Br | | |
| II-16 | 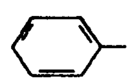 | $CH_3$ | $CH_3$ | S | $C-CH_3$ | | |

**Tabelle 2:** Beispiele für die erfindungsgemäß herzustellenden Verbindungen der Formel (II) (Fortsetzung)

| Beisp.-Nr. | $R^1$ | $R^2$ | $R^3$ | X | Y | Ausbeute (% d.Th.) | Schmelzpunkt (°C) |
|---|---|---|---|---|---|---|---|
| II-17 | 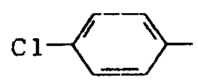 | $CH_3$ | $CH_3$ | S | N | | |
| II-18 | 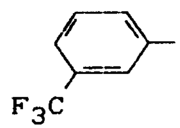 | $CH_3$ | $CH_3$ | S | N | | |
| II-19 | 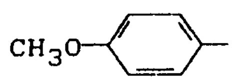 | $CH_3$ | $CH_3$ | S | CH | 96 | 87 |
| II-20 | 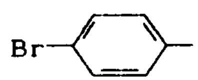 | $CH_3$ | $CH_3$ | S | N | | |

Verbindungen der Formel (VI):

Beispiel VI-1

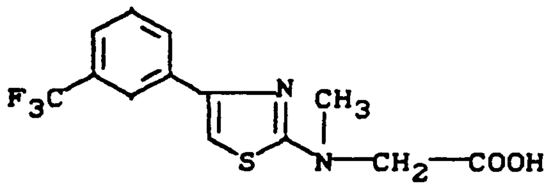

Zu einer Mischung aus 77,5 g (0,3 Mol) 2-Methylamino-4-(3-trifluormethyl-phenyl)-thiazol, 43,7 g (0,375 Mol) Chloressigsäure-Natriumsalz und 240 ml N,N-Dimethylformamid gibt man innerhalb einer Stunde portionsweise 46 g (0,41 Mol) Kalium-tert-butanolat zu. Durch leichte Kühlung wird dabei die Temperatur der Reaktionsmischung auf ca. 30 ° C gehalten. Man rührt 5 Stunden bei Raumtemperatur nach und gießt dann die Mischung in 1,5 l Wasser. Dann wird 3 mal mit je 100 ml Methylenchlorid ausgeschüttelt. Die wäßrige Phase wird mit Salzsäure versetzt und auf pH 2 eingestellt. Das ausgefallene Produkt wird abgesaugt, mit kaltem Wasser nachgewaschen und bei 50 ° C im Vakuum getrocknet. Man erhält auf diese Weise 86,4 g (91 % der Theorie) N-Methyl-N-(4-(3-trifluormethyl-phenyl)-thiazol-2-yl)-aminoessigsäure vom Schmelzpunkt 155 ° C.

Analog Beispiel VI-1 können beispielsweise auch die in der nachstehenden Tabelle 3 aufgeführten Verbindungen der Formel (VI) hergestellt werden.

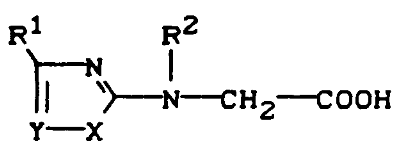

(VI)

**Tabelle 3:** Beispiele für die erfindungsgemäß herzustellenden Verbindungen der Formel (VI)

| Beisp.-Nr. | $R^1$ | $R^2$ | X | Y | Ausbeute (% d.Th.) | Schmelzpunkt (°C) |
|---|---|---|---|---|---|---|
| VI-2 | 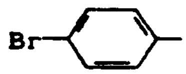 | $CH_3$ | S | CH | 93 | 151 |
| VI-3 | 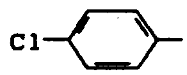 | $CH_3$ | S | CH | 79 | 156 |
| VI-4 | 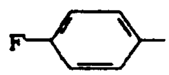 | $CH_3$ | S | CH | | |
| VI-5 | 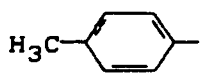 | $CH_3$ | S | CH | 84 | 149 |
| VI-6 | 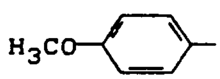 | $CH_3$ | S | CH | 85 | 100 |
| VI-7 | 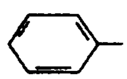 | $CH_3$ | S | CH | 77 | 133 |
| VI-8 | 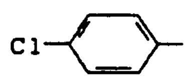 | $C_2H_5$ | S | CH | | |
| VI-9 | 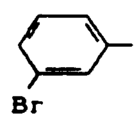 | $CH_3$ | S | CH | 98 | 146 |
| VI-10 | 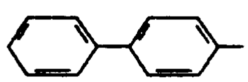 | $CH_3$ | S | CH | | |

Tabelle 3: Beispiele für die erfindungsgemäß herzustellenden Verbindungen der Formel (VI) (Fortsetzung)

| Beisp.-Nr. | $R^1$ | $R^2$ | X | Y | Ausbeute (% d.Th.) | Schmelzpunkt (°C) |
|---|---|---|---|---|---|---|
| VI-11 | 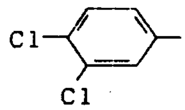 | $CH_3$ | S | CH | | |
| VI-12 | 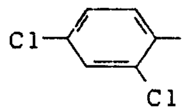 | $CH_3$ | S | CH | | |
| VI-13 | 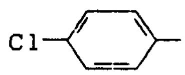 | $CH_3$ | S | N | | |
| VI-14 | 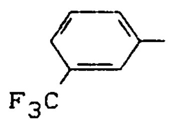 | $CH_3$ | S | CH | | |
| VI-15 | 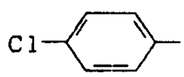 | $CH_3$ | S | C-Cl | | |
| VI-16 | 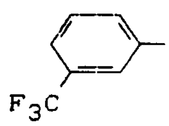 | $CH_3$ | S | C-Br | | |
| VI-17 | 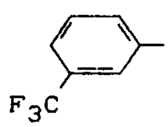 | $CH_3$ | S | N | | |
| VI-18 | 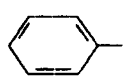 | $CH_3$ | S | $C-CH_3$ | | |
| VI-19 | 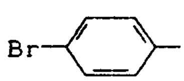 | $CH_3$ | S | N | | |

Verbindungen der Formel (VIII)

Beispiel VIII-1

Stufe 1

1) p-Brombenzamidin-Hydrochlorid

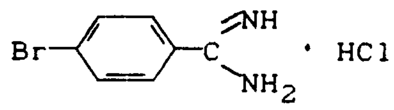

Ein Gemisch aus 182 g (1 Mol) 4-Brombenzonitril, 200 ml Methanol, 54 g (1 Mol) Ammoniumchlorid und 64 ml (2 Mol) flüssiges Ammoniak wurde 18 Stunden in einem 0,7 l V2A Rührautoklaven auf 125°C erhitzt. Das erhaltene Salz wurde abgesaugt, mit Di-isopropylether gewaschen und getrocknet. Es wurden 203 g 4-Brombenzamidin-Hydrochlorid mit einem Gehalt nach HPLC von 99,6 % und einer Ausbeute von 86 % der Theorie erhalten.

Stufe 2

2) N-(p-Brom-benzimidoyl)-N'-methylthioharnstoff

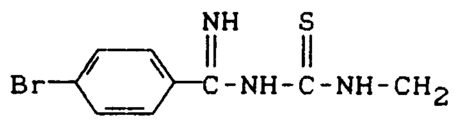

Zu 117,8 g (0,5 Mol) p-Brombenzamidin-Hydrochlorid in 360 ml warmen Wasser wurde zuerst bei Raumtemperatur eine Lösung aus 38 g (0,5 Mol) Methylsenföl und 120 ml Ethanol, dann eine Natronlauge aus 20,8 g Natriumhydrochlorid und 100 ml Wasser getropft. Dabei fiel unter Erwärmung auf 30-32°C ein Feststoff aus. Das Reaktionsgemisch wurde über Nacht bei Raumtemperatur nachgerührt, der Feststoff abgesaugt und mit Wasser gewaschen. Nach dem Trocknen wurden 109 g eines farblosen Feststoffs mit einem Schmelzpunkt von 129°C aus Cyclohexan/Ethanol umkristallisiert und einer Ausbeute von 80 % der Theorie erhalten.

Stufe 3

3) 5-Methylamino-3-(4-bromphenyl)-1,2,4-thiadiazol

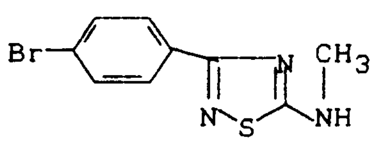

In eine siedende Suspension aus 100 g (0,37 Mol) N-(p-Brombenzimidoyl) N'-methylthioharnstoff und 400 ml Ethanol wurde langsam eine Lösung aus 110 g (1,13 Mol) Wasserstoffperoxid, 35 % und 300 ml Wasser zugetropft. Das Reaktionsgemisch wurde 15 Stunden unter Sieden nachgerührt, abgekühlt und der Feststoff abgesaugt und mit Wasser gewaschen. Nach dem Trocknen wurden 90,8 g einer farblosen kristallinen Substanz mit einem Schmelzpunkt von 172°C und einer Ausbeute von 90 % der Theorie erhalten.

Die in Tabelle 1 als Beispiel Nr. I-7 aufgeführte Verbindung kann beispielsweise wie folgt hergestellt werden:

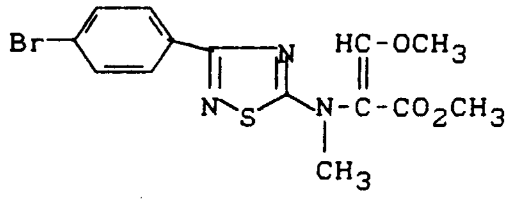

Einer Lösung aus 27 g (0,07 Mol) 5-(N-Methyl-N-carbomethoxymethylamino-3,4-bromphenyl)-1,2,4-thiadiazol in 80 ml Dimethylformamid und 93 g (1,55 Mol) Ameisensäuremethylester wurden portionsweise 9,6 g (0,17 Mol) festes Natriummethylat zugesetzt - Gemisch schäumte stark -. Der Reaktionsansatz wurde 18 Stunden zuerst bei 0-5 °C dann über Nacht bei Raumtemperatur nachgerührt und dann bei 0-5 °C mit 22,9 g (0,16 Mol) Dimethylsulfat versetzt. Nach einer Nachrührzeit von 2 Stunden bei Raumtemperatur wurde das Lösungsmittel im Vakuum abdestilliert. Der Rückstand wurde mit 5 % wäßriger Bicarbonat-Lösung und Petrolether verrührt. Das erhaltene Festprodukt wurde abgesaugt und getrocknet. Es wurden 26 g eines farblosen Pulvers mit einem Gehalt nach HPLC von 89,6 % Z-Isomeres und 9,8 % E-Isomeres, entsprechend einer Ausbeute von 95 % der Theorie erhalten.

Die in Tabelle 2 als Beispiel Nr. II-20 aufgeführte Verbindung kann beispielsweise wie folgt hergestellt werden:

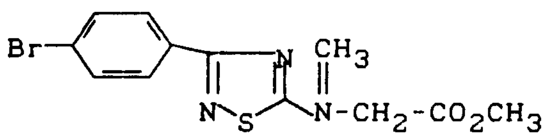

In einer Lösung von 6,8 g, 0,025 Mol, 5-Methylamino-3-/4-bromphenyl)-1,2,4-thiadiazol in 40 ml Dimethylsulfoxid wurden zuerst 2 g (0,03 Mol) Kaliumhydroxidpulver, 88 %ig, gelöst und dann dazu unter Kühlung 3,3 g (0,03 Mol) Chloressigsäuremethylester bei 15-20 °C getropft. Das Reaktionsgemisch wurde 3 Stunden bei Raumtemperatur nachgerührt, auf Wasser geschüttet, der Feststoff abgesaugt und mit kaltem Wasser gewaschen. Der getrocknete Stoff 8,4 g, hatte nach HPLC einen Gehalt von 91 % entsprechend einer Ausbeute von 90 % der Theorie.

## Patentansprüche

**1.** Verfahren zur Herstellung von 3-Alkoxy-2-heteroazolylamino-acrylsäureestern der allgemeinen Formel (I)

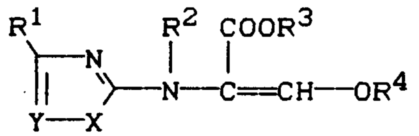

(I)

in welcher

- $R^1$ für jeweils gegebenenfalls substituiertes Aryl oder Heteroaryl steht,
- $R^2$ für Wasserstoff oder Alkyl steht,
- $R^3$ für Alkyl steht,
- $R^4$ für Alkyl steht,
- X für Sauerstoff oder Schwefel steht und
- Y für Stickstoff oder die Gruppierung C-$R^5$ steht, wobei
- $R^5$ für Wasserstoff, Halogen, Cyano, Formyl, Carboxy, Nitro oder für jeweils gegebenenfalls substituiertes Alkyl, Alkoxy oder Alkylthio oder für Alkoxycarbonyl steht,

dadurch gekennzeichnet daß man Heteroazolylaminoessigsäureester der allgemeinen Formel (II)

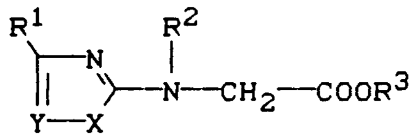

(II)

in welcher

$R^1$, $R^2$, $R^3$, X und Y die oben angegebene Bedeutung haben,

mit Ameisensäureestern der allgemeinen Formel (III)

$HCOOR^6$ (III)

in welcher
$R^6$ für Alkyl steht,
in Gegenwart eines Alkalimetallalkanolats und gegebenenfalls in Gegenwart eines Verdünnungsmittels bei Temperaturen von 0 ° C bis 100 ° C umsetzt und das hierbei erhältliche Produkt der Formel (IV)

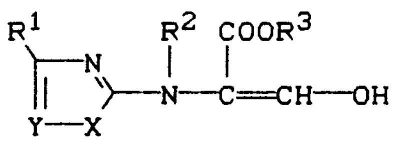

(IV)

in situ (ohne Zwischenisolierung) mit einem Alkylierungsmittel der allgemeinen Formel (V)

$X^1$—$R^4$ (V)

in welcher
$R^4$ die oben angegebene Bedeutung hat und
$X^1$ für Halogen oder die Gruppierung $-O-SO_2-O-R^4$ steht,
bei Temperaturen von 0 ° C bis 100 ° C umsetzt.

**2.** Verfahren gemäß Anspruch 1, dadurch gekennzeichnet, daß man 3-Alkoxy-2-heteroazolylamino-acrylsäureester der Formel (I) gemäß Anspruch 1 herstellt, in welcher
$R^1$ für jeweils gegebenenfalls substituiertes Aryl oder Heteroaryl mit jeweils bis zu 10 Kohlenstoffatomen und gegebenenfalls 1 bis 4 Stickstoffatomen und/ oder einem Sauerstoff- oder Schwefelatom als Heteroatom(en) steht, wobei als bevorzugte Substituenten ausgewählt sind: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Aryloxyalkyl oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomenund gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen - insbesondere Stickstoff, Sauerstoff und/ oder Schwefel - im Heteroarylteil und gegebenenfalls 1 bis 4 Kohlenstoffatomen im geradkettigen oder

verzweigten Alkylteil;

$R^2$ für Wasserstoff oder für geradkettiges oder verzweigtes Alkyl mit 1 bis 6 Kohlenstoffatomen steht,

$R^3$ für geradkettiges oder verzweigtes Alkyl mit 1 bis 6 Kohlenstoffatomen steht,

$R^4$ für geradkettiges oder verzweigtes Alkyl mit 1 bis 6 Kohlenstoffatomen steht,

X für Sauerstoff oder Schwefel steht und

Y für Stickstoff oder die Gruppierung C-$R^5$ steht, wobei

$R^5$ für Wasserstoff, Halogen, Cyano, Formyl, Carboxy, Nitro oder für jeweils geradkettiges oder verzweigtes Alkyl, Halogenalkyl, Alkoxy, Halogenalkoxy, Alkylthio oder Halogenalkylthio mit jeweils 1 bis 4 Kohlenstoffatomen und gegebenenfalls 1 bis 9 Halogenatomen oder für geradkettiges oder verzweigtes Alkoxycarbonyl mit bis zu 4 Kohlenstoffatomen steht.

**3.** Verfahren gemäß Anspruch 1, dadurch gekennzeichnet, daß man als Alkalimetallalkanolate Natrium- oder Kaliumalkanolate mit 1 bis 6 Kohlenstoffatomen im geradkettigen oder verzweigten Alkanolatrest einsetzt.

**4.** Verfahren gemäß Anspruch 1, dadurch gekennzeichnet, daß man bei Temperaturen von 0 ° C bis 100 ° C arbeitet.

**5.** Verfahren gemäß Anspruch 1, dadurch gekennzeichnet, daß man auf 1 Mol Heteroazolylaminoessigsäureester der Formel (II) 5 bis 50 Mol Ameisensäureester der Formel (III), 1 bis 4 Mol Alkalimetallalkanolat, und in der Folgestufe 1 bis 4 Mol Alkylierungsmittel der Formel (V) einsetzt.

**6.** Verfahren zur Herstellung von Heteroazolylaminoessigsäureestern der allgemeinen Formel (II)

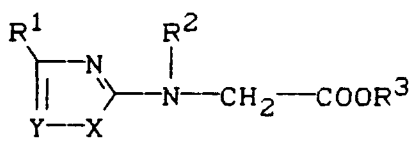

(II)

in welcher

$R^1$ für jeweils gegebenenfalls substituiertes Aryl oder Heteroaryl steht,

$R^2$ für Wasserstoff oder Alkyl steht,

$R^3$ für Alkyl steht,

X für Sauerstoff oder Schwefel steht und

Y für Stickstoff oder die Gruppierung C-$R^5$ steht, wobei

$R^5$ für Wasserstoff, Halogen, Cyano, Formyl, Carboxy, Nitro oder für jeweils gegebenenfalls substituiertes Alkyl, Alkoxy oder Alkylthio oder für Alkoxycarbonyl steht,

dadurch gekennzeichnet, daß man Heteroazolylaminoessigsäuren der allgemeinen Formel (VI)

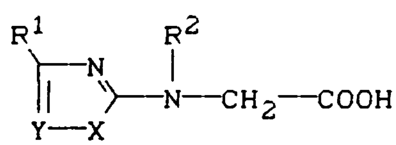

(VI)

in welcher

$R^1$, $R^2$, X und Y die oben angegebene Bedeutung haben,

mit Alkoholen der allgemeinen Formel (VII)

$HOR^3$(VII)

in welcher

$R^3$ die oben angegebene Bedeutung hat,

in Gegenwart saurer Veresterungskatalysatoren bei Temperaturen von 0 °C bis 120 °C umsetzt.

**7.** Heteroazolylaminoessigsäure der Formel (VI)

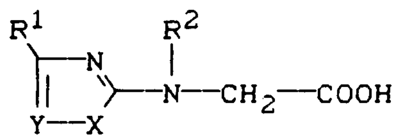

(VI)

in welcher

- $R^1$ für jeweils gegebenenfalls substituiertes Aryl oder Heteroaryl steht,
- $R^2$ für Wasserstoff oder Alkyl steht,
- X für Sauerstoff oder Schwefel steht und
- Y für Stickstoff oder die Gruppierung C-$R^5$ steht, wobei
- $R^5$ für Wasserstoff, Halogen, Cyano, Formyl, Carboxy, Nitro oder für jeweils gegebenenfalls substituiertes Alkyl, Alkoxy oder Alkylthio oder für Alkoxycarbonyl steht.

**8.** Verfahren zur Herstellung von Heteroazolylaminoessigsäuren der Formel (VI)

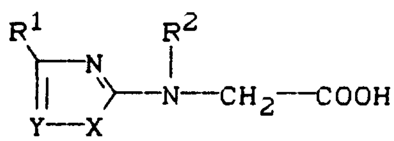

(VI)

in welcher

- $R^1$ für jeweils gegebenenfalls substituiertes Aryl oder Heteroaryl steht,
- $R^2$ für Wasserstoff oder Alkyl steht,
- X für Sauerstoff oder Schwefel steht und
- Y für Stickstoff oder die Gruppierung C-$R^5$ steht, wobei
- $R^5$ für Wasserstoff, Halogen, Cyano, Formyl, Carboxy, Nitro oder für jeweils gegebenenfalls substituiertes Alkyl, Alkoxy oder Alkylthio oder für Alkoxycarbonyl steht,

dadurch gekennzeichnet, daß man Aminoheteroazole der allgemeinen Formel (VIII)

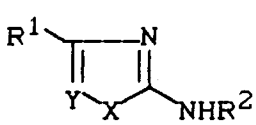

(VIII)

in welcher

$R^1$, $R^2$, X und Y die oben angegebene Bedeutung haben,

mit Halogenessigsäure-Salzen der allgemeinen Formel (IX)

$X^2—CH_2—COOM$ (IX)

in welcher

- M für ein Alkalimetall steht und
- $X^2$ für Halogen steht,

in Gegenwart eines Säureakzeptors und in Gegenwart eines Verdünnungsmittels bei Temperaturen von 0 °C bis 100 °C umsetzt.

**9.** Verfahren zur Herstellung von 3-Alkoxy-2-heteroazolylamino-acrylsäureestern der Formel (I) gemäß

Anspruch 1, dadurch gekennzeichnet, daß man ausgehend von den Aminoheteroazolen der Formel (VIII) gemäß Anspruch 8 die Verfahren gemäß den Ansprüchen 8, 6 und 1 kombiniert.

Europäisches Patentamt

# EUROPÄISCHER RECHERCHENBERICHT

Nummer der Anmeldung

EP 91113561.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.5) |
|---|---|---|---|
| D,A | EP - A1 - 0 389 901 (BAYER AG) * Ansprüche 1,6-14 * | 1,6-9 | C 07 D 277/46<br>C 07 D 285/08<br>//A 01 N 43/78<br>A 01 N 43/82 |
| D,A | DE - A1 - 3 905 119 (BAYER AG) * Ansprüche 1,7,8,11,12 * | 1,6-9 | |

RECHERCHIERTE SACHGEBIETE (Int. Cl.5)

C 07 D 277/00
C 07 D 285/00

Der vorliegende Recherchenbericht wurde für alle Patentansprüche erstellt.

| Recherchenort | Abschlußdatum der Recherche | Prüfer |
|---|---|---|
| WIEN | 30-09-1991 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82